(19) Europäisches Patentamt
European Patent Office
Office européen des brevets

(11) Publication number: 0 147 222
A2

(12) **EUROPEAN PATENT APPLICATION**

(21) Application number: 84309063.0

(22) Date of filing: 21.12.84

(51) Int. Cl.[4]: **A 01 N 47/18**
A 01 N 43/78, A 01 N 43/32
A 01 N 35/02, A 01 N 31/08
//(A01N47/18, 43:32, 35:02, 31:08, 31:04, 31:02), (A01N43/78, 43:32, 35:02, 31:08, 31:04, 31:02), (A01N43/32, 31:04, 31:02), (A01N35/02, 31:04, 31:02), (A01N31/08, 31:04)

(30) Priority: 23.12.83 GB 8334422

(43) Date of publication of application: 03.07.85 Bulletin 85/27

(84) Designated Contracting States: AT BE CH DE FR IT LI LU NL SE

(71) Applicant: STERWIN AG.
Zeughausgasse 9
CH-6300 Zug(CH)

(72) Inventor: Brocklehurst, Peter
87 Cowley Lane
Chapeltown Sheffield S30 4SX(GB)

(72) Inventor: Scaife, Rodney
4 Habershon Drive
Chapeltown Sheffield S30 4ZT(GB)

(74) Representative: Green, Alan James et al,
Sanderson & Co. 97 High Street
Colchester Essex C01 1TH(GB)

(54) **Composition for use in preserving hydrocarbon products.**

(57) The invention relates to a composition for use in preserving hydrocarbon products such as aviation and marine fuel oils.

The composition is based on an organic biocide containing carbon, hydrogen and oxygen only or a mixture of two or more such biocides, and at least one hydroperoxide of the formula:

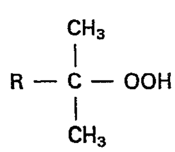

I

wherein R is an aliphatic or aromatic hydrocarbon group, optionally together with a carrier solvent which is compatible with the product to be preserved. Also, in the case of compositions for use in preserving marine fuel oils, they may include a small amount (usually less than 0.1% by weight) of a biocide containing nitrogen and/or sulphur in addition to said hydroperoxide and said organic biocide containing carbon, hydrogen and oxygen only.

EP 0 147 222 A2

Croydon Printing Company Ltd.

# COMPOSITION FOR USE IN PRESERVING HYDROCARBON PRODUCTS

The present invention relates to a composition for use in preserving hydrocarbon products, in particular liquid products such as kerosene and other fuel oils.

In the fuel oil industry there is increasing concern over problems resulting from microbiological contamination. While an aviation fuel oil is permitted by current aviation standards to contain no more than 30 ppm of water, the presence of even such small amounts of water can permit microbiological growth to occur. Thus, for example, in the case of oil filter coalescers associated with oil feed lines, these can become saturated with water and can thereby provide a site at which microbiological growth can take place. Furthermore, this growth can take place to such an extent that corrosion can occur, the filters become clogged, and the fuel oil line blocked. Although in some instances the results of this blocking may not be serious, in the case of say a fuel line to an aircraft engine, the results can be undesirable in the extreme. Also, infected coalescers can provide a source of subsequent contamination, even after a blockage has been cleared.

Also, while a marine fuel oil can contain higher levels of water (which can be introduced accidentally or deliberately to the fuel), such water again partitions and microbiological growth is common at the water/fuel interface. Usually in marine fuel oils contamination is caused by growth of the fungus Cladosporium resinae, and in use of such oils contamination can again cause filter blockages, coalescer blockages and corrosion problems.

We have now found surprisingly that a combination of certain tertiary alkyl or mixed alkyl/aryl hydroperoxides, together with small amounts of one or more other biocides containing only carbon, hydrogen and oxygen, can provide a synergistic preservative effect in such hydrocarbon products, and prevent degradation due to microbial growth. We have also found that where the combination is to be used to preserve marine fuel oils a further biocide containing nitrogen and/or sulphur may be provided at a low level and, in particular, that certain agricultural fungicides when used in very small amounts can enhance the synergism between the

hydroperoxide and any biocide containing only carbon, hydrogen and oxygen.

Accordingly, the present invention provides a composition for use in preserving hydrocarbon products such as aviation and marine fuel oils, which composition comprises an organic biocide containing carbon, hydrogen and oxygen only or a mixture of two or more such biocides, and at least one hydroperoxide of the formula:

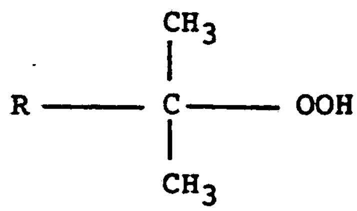

I

wherein R is an aliphatic or aromatic hydrocarbon group, optionally together with a carrier solvent which is compatible with the product to be preserved.

In the composition of the invention the said organic biocide should contain no nitrogen, sulphur or halogens, and generally must be a compound or mixture of compounds containing carbon, hydrogen and oxygen only. It must also be completely combustible giving rise to no residues or strongly acidic oxides or other acidic material apart from oxides of carbon. However, these criteria having been met the organic biocide may be any such biocide, although it is preferably an aldehyde such as formaldehyde (including compounds which release formaldehyde) or glyoxal, a dioxane such as a dimethoxane, and/or a phenol, including phenol itself, naphthols and indanols.

More preferably the or one of the organic biocides is a phenol of the formula:

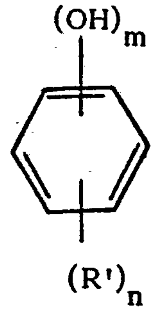

II

wherein m and n are the same or different and are each 1, 2 or 3 and R' is hydrogen, alkyl, alkoxy, hydroxyalkyl, aryl, alkaryl or aralkyl.

In the case where the organic biocide is a phenol of formula II and R' is alkyl, alkoxy or hydroxyalkyl, any such group is preferably one containing from 1 to 7 carbon atoms. Preferably, however, R' is alkyl, more preferably $C_1$ to $C_7$ alkyl, or aryl and m is 1. Thus, the preferred compositions of the invention may contain one or more alkyl phenols such as one or more of a heptyl phenol, a propyl phenol, o-, m- or p-cresol or a xylenol or an aryl phenol such as o-phenylphenol. An example of a propyl phenol biocide useful in the present invention is that sold commercially as a mixture of propylated phenols.

Preferably, the composition of the invention is in liquid form. In that case, the biocide may be selected so that it provides a carrier solvent for the hydroperoxide of formula I or vice versa. Thus, for example, a biocidal solvent may be provided by employing one or more liquid phenols such as a cresol or xylenol. Alternatively, or even additionally, a liquid carrier solvent which itself is a hydrocarbon product may be employed. In that case the solvent may be an aliphatic or aromatic hydrocarbon solvent, and typically may be a kerosene such as ECS 2012 sold by Esso or a commercially available aviation fuel.

In the composition of the invention, the hydroperoxide of formula I is preferably tertiary butyl hydroperoxide (R=methyl), tertiary amyl hydroperoxide (R=ethyl) and/or cumene hydroperoxide (R=phenyl). Most preferably, the hydroperoxide is tertiary butyl hydroperoxide for aviation fuel preservative compositions, and cumene hydroperoxide for other hydrocarbon fuel, e.g. marine fuel, preservative compositions.

The composition of the invention generally may comprise any compatible mixture of the biocide and the hydroperoxide. Typically, however, a composition in accordance with the invention will comprise up to about 30% by weight of organic biocide and up to about 70% by weight of hydroperoxide of formula I.

As a lower limit it may be said that the composition will generally include at least about 0.1%

by weight of organic biocide, a preferred range being from about 0.1% to about 5% by weight of organic biocide. In addition, the composition will usually include at least about 5%, typically from about 5% to about 60%, by weight of hydroperoxide of formula I, a preferred range being from about 5% to about 30% by weight of hydroperoxide of formula I.

Conveniently, the weight ratio of organic biocide to hydroperoxide of formula I may be up to about 1:5. Preferably, however, the weight ratio is much lower, typically at least about 1:10, and more preferably is about 1:40. In a particularly preferred aspect, the composition for use in preserving fuel oils in accordance with the invention comprises from about 0.5% to about 2% or about 3% by weight of an alkylated phenol, together with from about 15% to about 20% or about 30% by weight of tertiary butyl or cumene hydroperoxide, in a fuel oil carrier solvent.

In the composition of the invention, the ingredients of the composition other than hydroperoxide and the said biocide should be chosen so that they do not introduce into the fuel oil any unacceptable contamination. Thus, generally such ingredients will be free from halogens and free from or sufficiently low in nitrogen and sulphur to permit any necessary local regulations to be met. In the case of aviation fuels such regulations generally will be more stringent than say in the case of marine fuels, and usually will specify nitrogen- and sulphur-free requirements.

Nevertheless, and particularly in the case of a marine fuel preservative, it is one preferred aspect of the invention to provide a composition including a nitrogen and/or sulphur containing biocide, typically a fungicide, in addition to the hydroperoxide and the said organic biocide. Such a biocide may be included at very low levels, typically of less than 0.1% by weight e.g. about 100 ppm or less, and is preferably selected from the class of benzimidazole agricultural fungicides. Thus, for example, the additional biocide may be:

Benomyl, that is methyl-1-(butylcarbamoyl)-2-benzimidazole-carbamate;

Carbendazim, that is methyl-2-benzimidazole-carbamate; and/or

Thiabendazol, that is 2-(4-thiazolyl)-1H-

benzimidazole.

Such a fungicide when used at the above-defined levels may be employed with say about 30% by weight of peroxide, about 2% to about 3% by weight of biocide e.g. a phenol, and with the balance of the composition being a liquid carrier solvent.

The composition of the invention is employed as a preservative by adding a suitable amount of the composition to a hydrocarbon product, such as a fuel oil, for example, in situ in a storage tank. While the fuel oil is in the tank situation, the ingredients of the composition will work at the water/hydrocarbon interface to control and/or eliminate undesirable microorganisms.

The composition of the invention may be employed at dilutions in the product to be protected down to about 1 ppm. Alternatively, where say a storage tank is to be cleaned or sterilized, dilutions up to about 1% by weight may be employed. Generally, however, for control of microorganisms within a bulk hydrocarbon product the composition will be used at dilutions in the range of from about 1 ppm to about 500 ppm, preferably from about 10 to about 100 ppm. On the other hand, for problem situations the use dilution will be from about 0.1% to about 0.2%, and for tank cleaning also it is preferred to employ a dilution of about 0.2% by weight.

The compositions of the invention are illustrated by the following specific Examples.

Since tertiary butyl hydroperoxide as used in the invention is conventionally supplied as a 70% solution in water, the compositions of the invention may be blended by the following process technique:

1. The aqueous tertiary butyl hydroperoxide solution is added to the carrier solvent to produce a two phase system.
2. The water phase is removed by a suitable separation technique e.g. by the use of a separating funnel, leaving the hydroperoxide dissolved in the carrier solvent.
3. The organic biocide(s) is added to the carrier solvent plus hydroperoxide, and the final mixture is either filtered or passed through a coalescer to remove any undissolved solids or residual water.

(Where the peroxide is not supplied as a 70% solution in water the separation step is omitted).

## Example 1

A preservative composition was formulated by blending as follows:

| Ingredients | Percent by weight |
|---|---|
| A mixture of xylenols | 0.5 |
| Tertiary butyl hydroperoxide (100%) | 20 |
| ECS 2012 (a kerosene containing 17% aromatics and sold by Esso) | balance to 100% |

## Example 2

A preservative composition was formulated by blending as follows:

| Ingredients | Percent by weight |
|---|---|
| A mixture of xylenols | 2.0 |
| Tertiary butyl hydroperoxide (100%) | 20 |
| ECS 2012 | balance to 100% |

Example 3

A preservative composition was formulated by blending as follows:

| Ingredients | Percent by weight |
|---|---|
| A mixture of propylated phenols | 0.5 |
| Tertiary butyl hydroperoxide (100%) | 20 |
| ECS 2012 | balance to 100% |

Example 4

A preservative composition was formulated by blending as follows:

| Ingredients | Percent by weight |
|---|---|
| o-Phenylphenol | 1.6 |
| Tertiary butyl hydroperoxide (100%) | 15.6 |
| ECS 2012 | 82.8 |

The above composition is useful both as an aviation fuel preservative and as a marine fuel preservative.

As an aviation fuel preservative it can be employed at the following use dilutions, namely:

1. At the point of manufacture of the fuel oil - 100 ppm.
2. For problem situations - 0.1% to 0.2% v/v.

The composition has been tested in the laboratory to determine whether it adversely affects aviation fuel with respect to the AVTUR FII standard. To date all results have shown that the preservative causes no significant changes.

The preservative prevents growth of fungi or bacteria in the fuel or in a 10% v/v "water bottom" containing $\geqslant 10^6$ organisms per ml. (It is expected, however, that there will be no significant "water bottoms" in practical situations due to the effort put in by the Fuel Companies to reduce the water content in aviation fuels to less than 30 ppm. Accordingly, these results indicate that under the severest of conditions, much more severe than those ever likely to be encountered, the biocide is effective.)

As a marine fuel preservative the composition can be employed at a use dilution for problem situations of from 0.1% to 0.2% v/v. Tests to date show no adverse effects on the fuel, for example, no emulsions are

formed. The preservative is effective in the presence of simulated sea water and has a similar spectrum of activity as in the aviation fuel situation. It is expected that as much as 10% "water bottoms" will collect in marine fuel situations and a preservative for use therein must be able to cope with such situations. As indicated above the present preservative prevents growth of fungi or bacteria in a 10% v/v "water bottom".

## Example 5

A marine fuel oil preservative composition was formulated by blending as follows:

| Ingredients | Percent by weight |
|---|---|
| A mixture of propylated phenols | 1.6 |
| Tertiary butyl hydroperoxide (100%) | 16 |
| Carbendzazim, that is methyl-2-benzimidazole-carbamate | < 0.01 |
| ECS 2012 | balance to 100% |

## Examples 6 to 10

Each of the above Examples 1 to 5 was repeated except that the tertiary butyl hydroperoxide (100%) was replaced by cumene hydroperoxide (100%) to provide fuel oil preservative compositions especially suitable for marine fuel oils.

## Example 11

A marine fuel oil preservative composition was formulated by blending as follows:

| Ingredients | Percent by weight |
|---|---|
| A mixture of propylated phenols | 3 |
| Cumene hydroperoxide (100%) | 30 |
| Benomyl, that is methyl-1-(butylcarbamoyl)-2-benzimidazole-carbamate | < 0.01 |
| Kerosene | Balance to 100% |

Similar compositions may be prepared using tertiary butyl hydrogen peroxide and/or other phenolic biocides, as well as other fungicides, such as Carbendazim, that is methyl-2-benzimidazole-carbamate, and Thiabendazol, that is 2-(4-thiazolyl)-1H-

benzimidazole (at a level up to 0.1% by weight), with similar results.

The synergistic effect exhibited by compositions in accordance with the invention containing tertiary butyl hydroperoxide (TBHP) was demonstrated as follows:

The Minimum Inhibitory Concentrations for TBHP, organic biocide alone and mixtures of TBHP plus biocide against a single test organism was determined in an aqueous medium. The test organism chosen was <u>Pseudomonas aeruginosa</u> since this has previously been shown to be one of the organisms most resistant to TBHP systems. To enable the test to be carried out in water it was necessary to emulsify the compounds using a detergent (secondary alkyl sulphate). The results obtained are set out in Table 1 below:

<u>Table 1</u>

| Compound/ Composition | Minimum Inhibitory Concentration (ppm of compound/composition) |
|---|---|
| TBHP | 100 |
| Dimethoxane (6-acetoxy-2,4-dimethyl-m-dioxane) | 125 |
| Xylenols | 125 |
| Propylated phenols | 250 |
| o-Phenylphenol | 125 |
| o-Cresol | 156 |
| 1:1 w/w mix of TBHP plus dimethoxane | 62 |
| 2:1 mix with dimethoxane | 23 |
| 5:1 mix with dimethoxane | 18 |
| 10:1 mix with dimethoxane | 17 |
| 1:1 w/w mix of TBHP plus propylated phenols | 31 |
| 2:1 mix with propylated phenols | 23 |
| 5:1 mix with propylated phenols | 18 |
| 10:1 mix with propylated phenols | 17 |
| 1:1 w/w mix of TBHP plus xylenols | 75 |
| 2:1 mix with xylenols | 75 |
| 10:1 mix with xylenols | 55 |
| 20:1 mix with xylenols | 55 |
| 1:1 w/w mix of TBHP plus o-cresol | 100 |
| 2:1 mix with o-cresol | 75 |

Table 1 (contd.)

| Compound/ Composition | Minimum Inhibitory Concentration (ppm of compound/composition) |
|---|---|
| 5:1 mix with o-cresol | 62 |
| 10:1 mix with o-cresol | 44 |
| 1:1 w/w mix of TBHP plus o-phenylphenol | 31 |

As can be seen from the above results, where a mixture of say TBHP and dimethoxane is used to inhibit the growth of Pseudomonas the expected concentrations of a 1:1 ratio mix would be 50 ppm TBHP plus 63 ppm dimethoxane. However, the actual concentrations were 31 ppm TBHP plus 31 ppm dimethoxane, which is a clear demonstration of synergism. Similar results are exhibited for the remaining mixtures.

The above mixtures of 1:1 w/w TBHP to biocide have been evaluated in Kerosene and Avtur Derv 2494 Fuel, giving protection against up to a 30% by weight inoculum of mixed culture at a use concentration of 0.3% by weight in the fuel oil.

Other concentrations have been shown to give protection pro-rata i.e. 0.1% by weight preservative composition protected against a 10% by weight inoculum of mixed culture.

In addition, a fungicidal screening test was carried out to measure percentage inhibition of germination of Cladosporium resinae by a slide culture technique, as follows:

The compound or composition under test was dissolved in acetone (1 ml) and diluted to 10 ml with DIESO fuel (kerosene based solvent) which had been sterilized by filtration. This solution was further diluted with sterilized DIESO (0.5 ml to 25 ml) to give a stock preparation containing 100 ppm (w/v) of said compound or composition, and 0.2% (v/v) of acetone in DIESO.

To prepare a spore suspension, 2.5 ml of a modified Bushnell and Hass medium, previously sterilised by autoclaving, was inoculated with one drop of a suspension of Cladosporium resinae spores to give 1 x $10^6$ spores of Cladosporium resinae $ml^{-1}$. Three 0.01 ml drops of this suspension were evenly distributed between the steel plates of specially adapted microscope slides,

and a coverslip placed across the steel plates. The DIESO solutions of the compound or composition under test were run in under the coverslips using a pasteur pipette (approximately 0.33 ml), so that with the original total aqueous suspension of 0.03 ml, a 11:1 ratio of DIESO to water was obtained. The slides were placed in covered dishes and incubated at 30°C for 48 hours.

After incubation the slides were examined under a microscope for germination and growth of conidia of Cladosporium resinae at the DIESO-water interface. Fungicidal activity in the test compound or composition was shown by failure of the conidia to germinate, and the production of short bent or deformed germ tubes. In untreated controls, long branching vegetative hyphae and conidiophores of Cladosporium resinae were observed. Two counts were made from each drop, from which the % germination was calculated.

In the above "slide test" the following results were obtained:

| | | |
|---|---|---|
| Tertiary butyl hydroperoxide | at 100 ppm | - germination not prevented |
| Givgard DXN (2,4-dimethyl-6-acetoxy-1,3-dioxane | at 100 ppm | - germination not prevented |
| Tertiary butyl hydroperoxide + Givgard DN X in a 10:1 ratio | at 18 ppm | - germination prevented |

In our co-pending British Patent Application No. 83-34423 there is described and claimed a composition for use as a preservative or disinfectant, which composition comprises one or more organic biocides selected from phenols, aldehydes, heterocyclic compounds, quaternary ammonium compounds and cationic compounds, and at least one hydroperoxide of the formula:

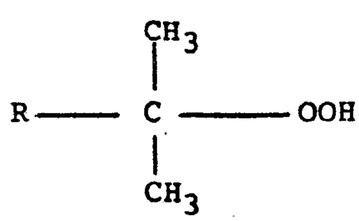

I

wherein R is an aliphatic or aromatic hydrocarbon group, in particular as an aqueous emulsion or dispersion, preferably one including a surface active agent present in an amount of at least about 10% by weight of the composition. Also, that other invention in a modification embraces a composition for use in preserving wood, which composition comprises at least one hydroperoxide of the formula I, at least one biocide and optionally a non-aqueous liquid carrier or solvent.

## CLAIMS

1. A composition for use in preserving hydrocarbon products such as aviation and marine fuel oils, which composition comprises an organic biocide containing carbon, hydrogen and oxygen only or a mixture of two or more such biocides, and at least one hydroperoxide of the formula:

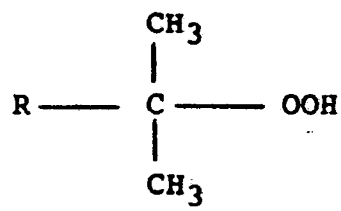

I

wherein R is an aliphatic or aromatic hydrocarbon group, optionally together with a carrier solvent which is compatible with the product to be preserved.

2. A composition according to claim 1, wherein the organic biocide is an aldehyde, a dioxane and/or a phenol including phenol itself, naphthols and indanols, such as a phenol of the formula:

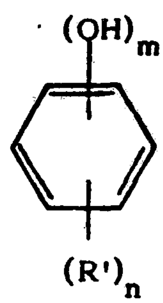

II

wherein m and n are the same or different and are each 1, 2 or 3 and R' is hydrogen, alkyl, alkoxy, hydroxyalkyl, aryl, alkaryl or aralkyl, and preferably one or more alkyl or aryl phenols.

3. A composition according to claim 2, wherein the phenol is one or more of a heptyl phenol, a propyl phenol, o-, m- or p-cresol, a xylenol or o-phenylphenol.

4. A composition according to any one of the preceding claims, wherein the composition is in liquid form and the biocide is selected so that it provides a carrier solvent for the hydroperoxide of formula I, the biocidal solvent preferably being a cresol, xylenol or

other liquid phenol.

5. A composition according to any one of claims 1 to 3, wherein the composition is in liquid form and there is provided a liquid carrier solvent which itself is a hydrocarbon product, such as kerosene.

6. A composition according to any one of the preceding claims, wherein the hydroperoxide of formula I is tertiary butyl hydroperoxide (R=methyl), tertiary amyl hydroperoxide (R=ethyl) or cumene hydroperoxide (R=phenyl).

7. A composition according to any one of the preceding claims, characterised in that the composition comprises up to about 30% by weight of organic biocide and up to about 70% by weight of hydroperoxide of formula I.

8. A composition according to claim 7, which includes at least about 0.1% by weight of organic biocide, preferably from about 0.1% to about 5% by weight of organic biocide, and at least about 5% by weight of hydroperoxide of formula I, preferably from about 5% to about 30% by weight of hydroperoxide.

9. A composition according to any one of the preceding claims, wherein the weight ratio of organic biocide to hydroperoxide of formula I is up to about 1:5, preferably about 1:10 or lower, e.g. about 1:40.

10. A composition according to any one of the preceding claims, wherein the composition also includes a small amount of a biocide containing nitrogen and/or sulphur in addition to said hydroperoxide and said organic biocide containing carbon, hydrogen and oxygen only, the additional biocide typically being present in an amount of less than about 0.1% by weight.

11. A composition according to claim 10, wherein the additional biocide comprises a benzimidazole compound, such as:

Benomyl, that is methyl-1-(butylcarbamoyl)-2-benzimidazole-carbamate;

Carbendazim, that is methyl-2-benzimidazole-carbamate; and/or

Thiabendazol, that is 2-(4-thiazolyl)-1H-benzimidazole.